# EUROPEAN PATENT SPECIFICATION

(11) **EP 1 996 176 B1**
(45) Date of publication and mention of the grant of the patent: **19.05.2010**
(21) Application number: 07751372.9
(22) Date of filing: 21.02.2007
(51) Int. Cl.: A61K 33/06, A61K 31/375, A61K 31/385, A61K 36/25

(54) **COMPOSITIONS TO REDUCE BLOOD GLUCOSE LEVELS AND TREAT DIABETES**
ZUSAMMENSETZUNGEN ZUR VERRINGERUNG DES BLUTGLUKOSESPIEGELS UND ZUR BEHANDLUNG VON DIABETES
COMPOSITIONS DESTINÉES À RÉDUIRE LES TAUX DE GLUCOSE SANGUIN ET À TRAITER LE DIABÈTE

(30) Priority: 21.02.2006 US 775188 P
(43) Date of publication of application: 03.12.2008
(73) Proprietor: Astrum Therapeutics Pty, Ltd., Melbourne VIC 3000 (AU)
(72) Inventor: HAYES, Eric, McMahons Point, New South Wales 2060 (AU); ZOLOTOY, Alexander, Richmond, British Columbia V6Y 1K2 (CA)
(74) Representative: Hatzmann, Martin
(86) International application number: PCT/US2007/004604
(87) International publication number: WO 2007/098240

(56) References cited:
- WO-A-00/57721
- WO-A-2006/110491
- CA-A1- 2 310 513
- US-A- 5 962 030
- US-A1- 2002 098 247
- ANONYMOUS: "Low Sugar" BOISECOOP, [Online] 2005, XP002467737 Retrieved from the Internet: URL:http://web.archive.org/web/20050505164 959/http://www.boisecoop.com/special-diets -low-sugar.asp> [retrieved on 2008-02-04]
- GUERRERO-ROMERO F ET AL: "Complementary Therapies for Diabetes: The Case for Chromium, Magnesium, and Antioxidants" ARCHIVES OF MEDICAL RESEARCH, INSTITUTO MEXICANO DEL SEGURO SOCIAL, MEXICO, MX, vol. 36, no. 3, May 2005 (2005-05), pages 250-257, XP004939732 ISSN: 0188-4409
- V. VUKSAN ET AL: "Konjac-Mannan and American Ginseng: Emerging Alternative Therapies for Type 2 Diabetes Mellitus" JOURNAL OF THE AMERICAN COLLEGE OF NUTRITION, vol. 20, no. 5, 2001, pages 370S-380S, XP002467738

## Description

### BACKGROUND OF THE INVENTION

### Field of the Invention

The present invention is related to compositions of natural agents that effectively reduce glucose levels in the blood of mammals, including humans. Composition 1 (Comp1) reinforces the efficacy of magnesium (Mg) salt against hyperglycemia. Composition 2 (Comp2) inhibits the formation of oxygen radicals produced by the insulin potentiating agent chromium picolinate (CrPic).

### Description of the Related Art

The prevalence of diabetes is increasing in developed countries as well as in developing countries as they become more affluent. Sixteen million Americans have diabetes, with an additional 798,000 new cases appearing annually. T2DM represents 80% of all diabetes in the USA and is increasing more rapidly in some ethnic groups (http://www.wrongdiagnosis.com/d/diab2/bastcs.htm; http://www.5mcc.com/Assets/SUMMARY/TP0264.htm)). The incidence of T2DM in non-Hispanic whites increased 300% in the decade between 1987 and 1996 (Burke J.P. et al., Arch Intern Med. 159:1450-1456, 1999). In Australia the prevalence of diabetes is estimated at 7.4% of the population whereas the prevalence of people exhibiting abnormal glucoses levels, and therefore at risk of developing diabetes, is 16.4%. The latter group increases sharply with age: 37% of those aged 55-64 years, 47% of those aged 65-74 years, and 53% of those aged 75 years and above. The prevalence of diabetes in Australia has more than doubled since 1981 and it is expected that 1.23 million Australians will suffer from diabetes in 2010 (Dunstan D.W., Diabetes Care 25:829-834, 2002; http://www.healthinsite.gov.au/content/internal/page.cfm?ObjID=00001FB3-0318-1D2D-81CF83032BFA006D). Approximately 1 in 4 Australians aged 25 years and older suffers from impaired glucose metabolism or clinical diabetes. The Australian and American government health regulatory agencies have stated that diabetes is one of the top health priorities (http://www.healthinsite.gov.au/content/internal/page.cfm?ObjID=00001FB3-0318-1D2D-81CF83032BFA006D). The combined population suffering from type 1 diabetes mellitus (T1DM) and T2DM in seven major world markets is projected to grow from 39.4 million to 49.4 million from 2003 to 2010, an estimated growth rate of 25.4% (http://www.ims-global.com/insight/news_story/0206/news_story_020611.htm).

T2DM causes numerous complications such as cardiovascular disease, stroke, blindness, nerve and renal damage and inflammatory disorders. In both human and economic terms, diabetes is one of the most costly diseases in the world. The American Diabetes Association (ADA) estimates that the total direct cost associated with diabetes care each year in the USA is US$ 45 billion and growing (Pardes H., Science 283:36-37, 1999). Indirect costs are very difficult to measure but a recent study in Sweden suggests that the indirect costs of treating diabetes are approximately 158% of the direct costs (Henriksson F., Pharmacoeconomics 20:43-53, 2002). Furthermore, complications arising from T2DM account for a 3.5-fold increase in individual costs and a 5.5-fold increase in hospitalization costs (Williams R. et al., Diaboteologia 47:S13-S17, 2002).

One of the main contributing factors of diabetes and diabetes related diseases is hyperglycemia (persistent abnormal elevation in blood glucose levels). There is a clear unmet medical need for effective and safe medications to reduce abnormal blood glucose profiles arid to treat and prevent diabetes and its ancillary pathology. In this regard, an extensive interest exists within clinical and research communities to develop natural products or compounds that are already present in mammals and act to reduce blood glucose levels. Despite the number of compositions proposed to be useful to treat T2DM, these do not guarantee safety and the majority of patents do not report efficacy or safety of the compositions as indicated in the following example. The composition of U.S. Patent 6,787,163 is proposed to regulate glucose levels and includes vanadyl sulfate, diethylethanolamine and copper. However, vanadyl sulfate demands a firm analysis with respect to cancer. The literature indicates cytotoxicity of vanadyl sulfate to cancer cells. However, there are reports of procancer activity of vanadyl sulfate with respect to DNA damage (Wozniak K., Arch Toxicol. 78:7-15, 2004), and of vanadium compounds promoting the induction of unwanted morphological transformation of hamster embryo cells (Rivedal E. et al., Cell Biol Toxicol. 6:303-314, 1990). Exposure of humans to diethanolamine results in irritation symptoms, and it contributes to the development of asthma in some (0.6%) individuals (Gadon M. E. et al., J. Occup. Med. 36(6):623-6, 1994). Administration of 100 ppm diethylethanolamine vapor to timed-pregnant Sprague-Dawley rats for 6 hours a day results in dry rales, statistically significant reduction of body weight (by 9.5%) and reduction of weight gain (by 48%) during exposure (Leiung H.V. et al., J Appl Toxicol. 18:191-196, 1998). Nasal cavities of rats exposed to 25 or 76 ppm of diethylethanolamine for 14 weeks revealed evidence of inflammatory cell infiltration, focal hyperplasia, and squamous metaplasia in the respiratory epithelium of the anterior nasal turbinate (Hinz J. P. et al., Fundam Appl Toxicol. 18(3):418-24, 1992). Copper is increased in T2DM patients and promotes heart failure. Copper chelation increases the level of copper in urine, increases copper excretion, reduces blood levels of copper and results in the alleviation of heart failure, improvement in cardiomyocyte structure and the reversal of left ventricular hypertrophy and collagen and beta(1) integrin deposition (Cooper G.J. et al. Diabetes 53: 2501-2508, 2004; US Patent 20030203973A1). Therefore, copper is expected to aggravate diabetes and its ancillary pathology.

There are two aspects of nutraceuticals that are important to consider with respect to diabetes: the efficacy of Mg salt and the safety of chromium picolinate as a hypoglycemic agent.

Indirect data have been reported to show a prominent effect of Mg in diabetes, wherein the level of consumption of Mg inversely correlated with the incidence of diabetes and use of Mg supplement resulted in a significant improvement in a lipid profile. However, seven clinical trials with the Mg supplement failed to demonstrate a reduction in blood glucose levels. This suggested that there were additional factors (from diet or as components of human body) that together with the Mg provided hyperglycemic effect in accordance with indirect data. These additional factors had not been identified.

There are also problems associated with chromium picolinate as a hyperglycemic agent. These include reports expressing concern over the safety of CrPic and studies failing to demonstrate its efficacy.

Thus there is a need: a) to identify agents that enhance the hypoglycemic effect of Mg, and b) to identify agents that reduce toxicity and may reinforce the hypoglycemic effect of CrPic.

### BRIEF SUMMARY OF THE INVENTION

Compositions Comp1 is provided. In certain embodiments, composition Comp1 comprises magnesium salt, konjac mannan, vitamin C, and alpha-lipoic acid. Also disclosed is composition Comp2 which comprises chromium picolinate, konjac mannan, vitamin C, and alpha-lipoic acid.

In certain embodiments, compositions Comp1 and/or Comp2 may further comprise an insulinogenic nutraceutical agent, American ginseng (AG), Uncaria tomentosa, Uncaria guianensis, and/or Urtica dioica. In certain such embodiments, the insulinogenic nutraceutical agent may be cinnamon extract. In certain other such embodiments, the insulinogenic nutraceutical agent may be, but is not limited to, gymnema sylvestre or aloe vera.

In certain embodiments, Comp1 is provided for use in a method for the prevention and/or treatment of diabetes and/or reduction of blood glucose levels in mammals, including humans, by administration of Comp1. In certain other embodiments, Comp1 may be used for the manufacture of a medicament for the prevention and/or treatment of diabetes and/or the reduction of blood glucose in mammals, including humans, by administration of Comp1. In further embodiments, Comp1 are provided for use in a method for the treatment and/or prevention of hyperglycemia, hyperinsulinemia, insulin resistance, oxidative stress, inflammation, and/or side effects associated with diabetes in mammals, including humans, by administration of Comp1 . In yet other embodiments, Comp1 may be used for the manufacture of a medicament for the treatment and/or prevention of hyperglycemia, hyperinsulinemia, insulin resistance, oxidative stress, inflammation, and/or side effects associated with diabetes in mammals, including humans, by administration of Comp1. Side effects of diabetes may include, but are not limited to, neuropathy, nephropathy, cardiomyopathy, stroke, and/or blindness.

In certain embodiments, Comp1 for use in methods described herein or for the manufacture of a medicament for use in methods described herein may potentiate hypoglycemic effects of a magnesium salt.

In certain embodiments, Comp 1 is provided for use in methods described herein or for the manufacture of a medicament for use in methods described herein wherein the amount of ingredients of Comp 1 administered comprises: magnesium chloride (1 to 3.5 g/day), alpha-lipoic acid (0.8 to 1.8 g/day), konjac mannan (3 to 8.6 g/day), Vitamin C (0.8 to 4 g/day), cinnamon extract (2 to 5 g/day), American ginseng (3 to 9 g/day), Uncaria tomentosa (0.35 to 2 g/day), Uncaria guianensis (0.35 to 2 g/day), Urtica dioica (0.25 to 1 g/day). In certain such embodiments, Comp 1 is provided for use in methods described herein or for the manufacture of a medicament for use in methods described herein wherein the amount of ingredients of Comp 1 administered comprises: magnesium chloride (2.5 g/day), alpha-lipoic acid (1.8 g/day), konjac mannan (3.6 g/day), Vitamin C (2 g/day), cinnamon extract (2 to 5 g/day), American ginseng (3 g/day), Uncaria tomentosa (0.8 g/day), Uncaria guianensis (0.8 g/day), Urtica dioica (0.5 g/day).

Also disclosed is Comp 2 wherein the amount of ingredients of Comp 2 administered comprises: chromium picolinate (1 to 6 mg/day), alpha-lipoic acid (0.8 to 1.8 g/day), konjac mannan (3 to 8.6 g/day), Vitamin C (0.8 to 4 g/day), cinnamon extract (2 to 5 g/day), American ginseng (3 to 9 g/day), Uncaria tomentosa (0.35 to 2 g/day), Uncaria guianensis (0.35 to 2 g/day), Urtica dioica (0.25 to 1 g/day). In certain such embodiments, Comp 2 is disclosed wherein the amount of ingredients of Comp 2 administered comprises: chromium picolinate (3.2 mg/day), alpha-lipoic acid (1.8 g/day), konjac mannan (3.6 g/day), Vitamin C (2 g/day), cinnamon extract (2 to 5 g/day), American ginseng (3 g/day), Uncaria tomentosa (0.8 g/day), Uncaria guianensis (0.8 g/day), Urtica dioica (0.5 g/day).

In certain embodiments, administration of Comp1 and/or Comp2 may be oral, parenteral, rectal, sublingual, or via inhalation. In some such embodiments, Comp1 and/or Comp2 may comprise a physiologically acceptable carrier suitable for such modes of administration.

Components of Comp1 and Comp2 are not associated with cellular, organ or systemic toxicity.

These and other aspects of the present invention will become apparent upon reference to the following detailed description.

### DETAILED DESCRIPTION OF THE INVENTION

The present invention discloses compositions that are surprisingly effective in reducing and stabilizing glucose levels in the blood of mammals, specifically in pre-diabetic patients and patients with type 2 diabetes mellitus (T2DM). Composition 1 (Comp1) reinforces the efficacy of Mg salt to reduce glucose levels by the combination of Mg salt with anti-inflammatory agents konjac-glucomannan (KGM), vitamin C and alpha-lipoic acid (α-LA). Composition 2 (Comp2) improves the safety of chromium picolinate (CrPic) by the combination of CrPic with anti-inflammatory agents KGM, vitamin C and α-LA to inhibit the formation of oxygen radicals.

### Inflammation and Diabetes

There is a positive relationship between inflammation and diabetes, as illustrated in the following examples. A positive correlation between insulin resistance and CRP level (r=0.83; p<0.001) was observed in non-diabetic patients (Abatecolla A.M. et al., J. Am. Geriatr. Sos. 52:399-404, 2004). T2DM patients exhibited an increased level of inflammatory mediators such as TNF-alpha, IL-6, IL-18 (Spranger J. et al., Diabetes 52:812-817, 2003; Desfaits A.C. et al., Diabetes Care 21: 487-493, 1998; Chang F.Y. et al., Diabetes Res Clin. Pract 28:137-146, 1995; Moriwaki Y. et al., Metabolism 52:605-608, 2003; Esposito K. et al., Am J Clin Nuts. 78:1135-1140, 2003). Hyperglycemia in humans (15 mmol/L for 5 hours) resulted in increased levels of TNF-alpha, IL-6 and IL-18. The effect was stronger and longer in subjects with impaired glucose tolerance versus controls (Esposito K. et al., Circulation 106(16):2067-72, 2002). Levels of these mediators, specifically IL-18, positively correlated with the level of urinary albumin excretion in patients with microalbuminuria, a surrogate marker of diabetic nephropathy (Moriwaki Y. *et al*., 2003). Diabetic monocytes collected from T2DM patients expressed a greater TNF-alpha mediated adhesion to endothelium (*e*.*g*., increase in sICAM and sE-selectin expression) compared to non-diabetic control subjects (Devaraj S. et al., Circulation 102:191-196, 2000; Kvasnicka J. et al., Sb Lek. 99:97-101, 1998). Release of IL-6 from the thigh during exercise positively correlated with glucose uptake into the thigh. (R=0.82, p=0.03) (Helge J. W. et al., J. Physiol. 546(1):299-305, 2003). Analogously the accumulation of IL-6 in adipocytes negatively correlated with glucose disposal *in vivo* (R=-0.75, p <0.02) and with insulin-stimulated glucose transport *in vitro* (R=-0.767, p<0.02) (Bastard J.P. et al., J Clin Endocrinol Metab 87:2084-2089, 2002). Increased expression of TNF-alpha was observed in adipose tissue of four diabetic rodent species: db/db, ob/ob, tub/tub, fa/fa. The prominent features of all four models were obesity and insulin resistance. Neutralization of TNF-alpha by a soluble human TNF-receptor-IgG fusion protein resulted in 2.5 times increase in the peripheral glucose disposal in obese insulin-resistant rats (Spiegelman B. M. et al., J. Biol. Chem. 268(10):6823-6826, 1993). Infusion of TNF-alpha into healthy patients completely inhibited insulin stimulated glucose uptake (Rask-Madsen C. et al., Circulation 108:1815-1821, 2003). This result was supported by the studies of Miyazaki Y. et al. (Int J Obes Relat Metab Disord. 27:88-94, 2003) which demonstrated a negative correlation (r = -0.45, p<0.01) between the level of TNF-alpha and insulin-stimulated glucose disposal and a positive correlation between the level of TNF-alpha and fasting glucose and insulin levels in healthy people and patients with impaired glucose tolerance. The same studies failed to find a correlation between TNF-alpha and insulin-stimulated glucose disposal in T2DM patients. Taken together, the observations described above indicate that TNF-alpha increases plasma glucose and insulin levels prior to the onset of type 2 diabetes. There was an inverse correlation reported between TNF-alpha and adiponectin (protein with anti-inflammatory properties) level in obese patients (Kem P.A. et al., Diabetes 52:1779-1785, 2003; Fernandez-Real J.M. et al., J Clin Endocrinol Metab 88:2714-27188, 2003). Decreased levels of adiponectin were clearly associated with T2DM (Daimon M. et al., Diabetes Care 26:2015-2020, 2003; Yang W.P. et al., J Clin Endocrinol Metab. 86: 3815-3819, 2001; Monzillo L.U. et al., Obes Revs. 11:1048-1054, 2003). Adiponectin correlated with insulin sensitivity (r = 0.67, p<0.001) (Kern *et al.,* 2003) and analysis of 1792 citizens of Japan resulted in the conclusion that low levels of adiponectin are associated with diabetes (p=0.009; Daimon M. *et al.,* 2003).

Thus, the application of anti-inflammatory compounds would be expected to result in a reduction in glucose levels. However the result varies from one compound to another. The anti-inflammatory glutathione reduces glucose levels. Anti-inflammatory Mg was highly advocated but a supplemental Mg salt failed in 7 prospective clinical studies of T2DM (see ref. below). Anti-inflammatory thalidomide and melatonin increase insulin resistance (Iqbal N. et al., Diabetes Care 23:1172-1176, 2000; Wilson J.S. et al., Lancet 2: 940-941 1966; Figg et al., Semin Oncol 28(4 Suppl. 15): 62-66, 2001; Clin Endocrinol (Oxf). 54(3):339-46, 2001).

### A Prominent Role of Mg in Diabetes

A cohort of 39,345 women >45 years without history of diabetes, cancer or cardiovascular disease was followed for 6 years. The cohort was divided by quintiles according to the consumption of magnesium. 918 cases of T2DM appeared during this time. The relative risk (RR) of diabetes had an inverse association with Mg consumption in a full cohort. The most significant effect was found for women with BMI ≥ 25 m². In this subgroup RR=1.0, 0.96, 0.76, 0.84, and 0.78 (P = 0.02 for trend) from the lowest to the highest quintile of Mg intake. Fasting insulin level displayed a negative correlation with Magnesium intake in healthy women with BMI≥ 25 m² (P<0.03) (Song Y. *et al., Diabetes Care 27*(1):59-65, 2004).

Retrospective analysis of 85,000 women and 43,000 men was conducted. After 18 years of follow-up in women and 12 years in men, the authors have documented the appearance of 4,085 and 1,333 incident cases of type 2 diabetes, respectively. It was found that the highest Mg uptake reduced the probability of diabetes to 0.66 and 0.67 accordingly compared to the subgroup with the lowest Mg uptake (P<0.001 for the trend) (R. Lopez-Ridaura et al., Diabetes Care 27(1):134-40, 2004).

Numerous publications have reported statistically significant correlations in humans with respect to the beneficial effects of Mg as measured for certain metabolic parameters, with r from -0.3 to -0.7 for plasma Mg compared to fasting glucose, HbA1C and insulin level (Alzaid A. et al., J. Clin. Endocrin. Metabol. 80:1376-81, 1996; Mikhail N. et al., South Med J. 92(12):1162-6, 1999; Schlienger J.L. et al., Presse Med. 17(21):1076-9, 1988; Fort P. et al., J Am Coll Nutr. 5(1):69-78, 1986).

Insulin-induced increase in erythrocyte Mg levels positively correlated with glucose disposal (r from 0.7 to 0.9; G. Paotisso et al., Diabetologia 1988, 31, 910-915; Paolisso G. et al., Diabete & Metabolism 16:328-33,1990; Paolisso G. et al., Am. J. Hypertens. 681-86, 1992).

In addition Magnesium significantly improves lipid profile. For example, Mg exhibited a negative correlation to total cholesterol (Srivastava V. et al., Ind J Med Sci. 47:119-123, 1993), triglycerides (Delva P. et al., Life Sci. 62:2231-2240, 1998), free fatty acid (Iskra M. et al., J Trace Elem Electrolytes Health Dis. 7:185-188, 1993) and a positive correlation to HDL cholesterol (Nozue T. et al., Magnes Res. 12:297-301, 1999; Zidek V. et a/., Med Wochenschr. 112:1787-1789, 1982). Magnesium supplement resulted in increases in HDL levels and reduction of triglycerides, LDL and total cholesterol in humans (Lal J. et al., J Assoc Phys Ind. 51:37-42, 2003; Itoh K. et al., Br J Nutr. 78:737-50, 1997; Corica F. et al., Magnes Res. 7:43-47, 1994).

Despite a prominent role of Mg in diabetes, seven clinical trials with the supplemental Mg had failed to show reduced glucose levels (Lima M.L. et al., Diabetes Care 21:682-686, 1994; de Valk H. W. et al., Diabetic Medicine 15:503-507, 1998; Gullestad et al., Diabetes Care 17(5):460-61, 1994; Johnsen S.P. et al., Ugeskr Laeger. 161(7):945-8, 1999; Eibl N.L. et al., Diabetes Care 18(2):188-192, 1995; Lal J. et al., J Assoc Physicians India. 1:37-42, 2003; Eriksson J. et al., Ann Nutr Metab. 39(4):217-23, 1995).

Thus it was evident that there are cofactors (from diet or as components of human body) that together with Mg yield the hypoglycemic effect and are necessary for the important role of magnesium in diabetes. These cofactors had not been identified.

Pharmacologically, there are two main roles of Mg: a) as a cofactor for the addition/elimination of phosphate group in biochemical reactions, and b) as an anti-inflammatory agent. As an example, reduced dietary Mg was found to lead to increased TNF-alpha, Substance P, IL-6 and other inflammatory particles (Vernet P. et al., Biochim Biophys Acta. 1675(1-3):32-45, 2004; Rude R.K. et al., J Nutr. 134(1):79-85, 2004; Bussiere F.I. et al., Br J Nutr. 87(2):107-13, 2002; Malpuch-Brugere S. et al., Biochim Biophys Acta. 1501(2-3):91-8, 2000). Use of Mg supplements has resulted in a significant or partial inhibition of inflammation (Ueshima K. et al., Magnes Res. 16(2):120-6, 2003; Lee K.H. et al., Mol Endocrinol. 4(11):1671-8, 1990).

It is known that an increase in inflammation accompanies an increase in glucose level. It could be expected then that the anti-inflammatory effect of Mg would contribute to a decrease in glucose levels and that supplemental use of additional anti-inflammatory agents may further stimulate this effect. As shown in the Examples herein, alpha-lipoic acid (α-LA), vitamin C and konjac-glucomannan (KGM), complemented the effect of Mg against hyperglycemia and hyperinsulinemia.

### Ingredients of Comp1 and Comp2

Alpha-lipoic acrid (α-LA) is an anti-oxidant that exhibits multiple effects directly against diabetes *in vitro* and in some animal models *in vivo,* but shows a marginal efficacy for reduction of glucose level in humans.

α-LA was shown to significantly limit NF-kappaB activation (Suzuki *et al.,* 1992; Bierhaus A *et al.,* 1997; Hoffman *et al.,* 1998; Cohen M.P., J Lab Clin Med. 141:242-249, 2003) and to partially prevent monocyte adhesion to endothelial cells (Yorek *et al.,* 2002; Zhang W.J. et al., FASEB J. 15:2423-2432, 2001). It was further shown to reduce levels of TNF-alpha and IL-6 in cancer patients (Mantovani G et al., J Environ Pathol Toxicol Oncol. 22:17-28, 2003), to decrease levels of peroxides in DM patients by 34% (Borcea V. et al., Free Radic Biol Med. 26:1495-1500, 1999), and to display a number of anti-inflammatory properties, including increasing the level of cellular glutathione (Fuchs J. et al., Arzneimittelforschung. 43:1359-1362, 1993; Bunin A. et al., Vestn Oftalmol. 108:13-15, 1992; Han D. et al., Biofactors. 6:321-338, 1997). Administration of glutathione to patients with alcoholic liver disease resulted in a significant decrease in the level of TNF-alpha, II-6 and IL-8 (Pena L.R. et al., J Parenter Enteral Nutr. 23:1-6, 1999). The inhibition of glutathione depletion prevented NF-kappaB activation induced by TNF-alpha (Ide N. et al., J Nutr. 131:1020S-1026S, 2001).

In addition, α-LA increased the uptake of glucose into muscle (Rett K. et al., Diabetes. 45:S66-S69, 1996), increased insulin sensitivity by activating AMP-protein kinase in skeletal muscle (Woo Je Lee et al., Biochem. Biophys. Res. Commun. 332:885-891, 2005), increased the ratio of NAD+/NADH, thus stimulating glycolysis (Roy S. et al., Biochem Pharmacol. 53:393-399, 1997), and inhibited gluconeogenesis in liver of normal and diabetic rats (Khamaisi M. et al., Metabolism 48:504-510, 1999). It has been hypothesized that the anti-gluconeogenic effect of α-LA in liver may be attributed to a sequestration of intramitochondrial acetyl-CoA as lipoyl-CoA, bisnorlipoyl-CoA or tetranorlipoyl-CoA (Blumental S.A., Biochem J. 219:773-780, 1984). A reduction in acetyl-CoA would lead to decreased citrate levels and thus with the activation of phosphofructokinase to enhanced glycolysis. It has also been hypothesized that the α-LA mediated reduction of gluconeogenesis may be the result of an inhibitory effect on biotin-dependent carboxylase, which catalyses an initial rate-limiting step in gluconeogenesis (Zemleni, J. Am J Clin Nutr. 65:508, 1997).

Despite a prominent role of α-LA *in vitro* and in some *in vivo* animal studies, the effect on glucose level in humans is weak. Two clinical trials reported reduction of fasting glucose level by 18% in patients with diabetic neuropathy (Rom, J Intern Med. 37(3):297-306, 1999) and by -15% in lean T2DM patients (Konrad T. et al., Diabetes Care 22:280-287, 1999). Two clinical trials showed no effect of lipoic acid on glucose levels (Hahm J.R. et al., J. Diab. Complic. 18:79-85, 2004; Evans J.L. et al., Endocr Pract. 8(1):29-35, 2002), while three studies reported no effect on fasting glucose level but claimed increase in insulin sensitivity by α-LA (Jacob S. et al., Free Radic Biol Med. 27(3-4):309-14, 1999; Jacob S. et al., Exp Clin Endocrinol Diabetes. 104(3):284-8, 1996; Jacob S. et al., Arzneimittelforschung. 45(8):872-4, 1995).

Vitamin C has been demonstrated to have numerous anti-inflammatory properties, such as reduction of CRP in active and passive smokers (Block G. et al., J Am Coll Nutr. 23(2):141-7, 2004), reduction of CRP and fibrinogen in patients with persistent atrial fibrillation (Korantzopoulos P. et al., Int. J. Cardiol.), and increase in the intracellular GSH level (Paolisso G. et al., J Am Coll Nutr. 14(4):387-92, 1995; Naziroglu M. et al., Clin Chim Acta. 344(1-2):63-71, 2004; Lenton K.J. et al., Am J Clin Nutr. 77(1):189-95, 2003).

Reported effects of vitamin C on glucose and insulin level have not been consistent. Most studies have demonstrated reduction of glucose level and increase in insulin sensitivity by vitamin C (Paolisso G. et a/., J Am Call Nutr., 14(4):387-92, 1995; Eriksson J. et al., Ann Nutr Metab. 39(4):217-23, 1995; Paolisso G. et al., Am J Physiol. 266(2 Pt 1):E261-8, 1994). Nevertheless, as a rule vitamin C has not been reported to affect control (Hirai N. et al., Amer. J. Physiol. Heart Circul. Physiol. 279(3):H1172-78, 2000; Hiroshima O. et al., J Am Coll Cardiol. 35(7):1860-6, 2000, Weykamp C.W. et al., Clin Chem. 41(5):713-6, 1995). Further, vitamin C has been reported to have no effect on T2DM and obese subjects (Martinez-Abundis E. et al., Rev Invest Clin. 53(6):505-10, 2001; Upritchard J.E. et al., Diabetes Care. 23(6):733-8, 2000).

Anti-inflammatory properties of konjac mannan have been reported to include reduction of apolipoprotoin B and apo B/A-I1 ratio (Vuksan V. et al., Diabetes Care. 23(1):9-14, 2000) and reduction of immunoreactive insulin (Doi K., Eur J Clin Nutr. 49:S190-S197, 1995).

Administration of konjac mannan with food has been reported to result in a generalized loss of weight (an average 2.75 kg), reduction of LDL and total cholesterol (-23% and -12.7%, respectively) and cholesterol in liver, and reduction of fasting and postprandial levels of glucose (on average -22%) (Huang Cheng-Yu et al., Biomed Environ Sci. 3:121-131, 1990; Walsh D.E. et al., Int J Obes. 8:289-293, 1984; Doi K. et a/., Lancet 1: 987-988, 1979; Doi K., Eur J Clin Nutr. 49:S190-S197, 1995; Chen Hsaio-Ling et al., J Am Coll Nutr. 22:36-42, 2003).

### Efficacy and Side Effects of Chromium Picolinate

Low molecular weight chromium binding substance (LMWCr), a protein that is present in all mammalian species, is stored in the cytosol of insulin-sensitive cells in an apo (unbound) form that is activated by binding four chromium III ions in a multinuclear assembly much like that of calmodulin (Vincent J.B., Acc Chem Res. 33:503-510, 2000). This activation is the result of a series of steps stimulated by insulin signaling. LMWCr has been shown to potentiate the action of insulin once insulin has bound to its receptor (Sun Y. et al., J Biol Inorg Chem. 5:129-136, 2000). This insulin potentiating or autoamplification action was reported to stem from the ability of LMWCr to maintain stimulation of tyrosine kinase activity (Anderson R.J., Am Coll Nutr. 17:548-555, 1998; Vincent, 2000). It has been further reported that, once insulin is bound to its receptor, LMWCr binds to the activated receptor on the inner side of the cell membrane and increases the insulin-activated protein kinase activity by eightfold (Davis C.M. et al., Arch Biochem Biophys. 339:335-343, 1997).

Data that support the effect of CrPic are described below. Thirty-nine T2DM patients with an average age of 73 years received 200 pg CrPic twice daily for three weeks and were compared to 39 age-matched controls. Changes versus baseline were as follows: reduction in glucose (-21%; p<0.001), glycated hemoglobin (HbA₁C) (from 8.2% to 7.6%; p<0.01), cholesterol (-9.4%; p<0.02) and triglycerides (-10.5%) (Rabinovitz H. et al., Int J Vitam Nutr Res. 74:178-182, 2004). T2DM patients receiving 200 µg CrPic twice daily for 12 weeks exhibited improvement in fasting glucose (0.44 mM; p<0.001), post-prandial serum glucose (1.97 mM; p<0.001) and a significant decrease in insulin level versus age-matched subjects receiving placebo control (Ghosh D. et al., J Nutr Biochem. 13:690-697, 2002). Review indicated significant reduction of glucose by CrPic in 11 out of 12 randomized clinical trials (Diabetes Educ. 2004; Suppl:2-14; editorial).

Side effects and in-consistency in reduction of glucose level have been reported with CrPic. Studies have reported an absence of efficacy of chromium in T2DM or IGT subjects (Gunton J.E. et al., Diabetes Care 28(3):712-3, 2005; Lee N.A. et al., Diabetes Care 17(12):1449-52, 1994; Abraham A.S. et al., Metabolism 41(7):768-71, 1992; Uusitupa M.I. et al., Br J Nutr. 68(1):209-16, 1992).

Reported side effects of CrPic in humans include anemia, thrombocytopenia, liver disfunction, renal failure (Ceruli J. et al., Ann. Pharmac. 32:428-31, 1998; Wasser W.G. et al., Ann. Intern. Med. 126:410, 1997), rhabdomyolysis (Martin W.R. et al., Pharmacotherapy 18:860-2, 1998), contact dermatitis (Fowler Jr. J.F., Cutis 65:116, 2000) and episodes of cognitive, perceptual, and motor changes (Mertz W. et al., Am J Physiol 209:489-494, 1965). *In vitro* studies have indicated mutagenic activity, mitochondrial damage and apoptosis in CHO cells by CrPic at supraphysiological concentration (0.2 - 1 mM) (Stearns D.M. et al., FASEB J 9:1643-1648, 1995; Bagchi D. et al., Res. Commun. Mol. Pathol. Pharmacol. 97:345-346, 1997; Bagchi D. et al., Toxicol. 180:5-22,2002; Stearns D.M. et al., Mutation Res. 513:135-142, 2002). In the presence of reducing agents at physiologically relevant concentrations (120 nM), CrPic produced hydroxyl radicals that nicked DNA (Speetjens J.K. et al., Chem Res Toxicol. 12:483-487, 1999). The formation of reactive oxygen radicals and oxidative damage by CrPic was supported by *in vivo* studies (Hepburn D.D.D. et al., Polyhedron 22:455-63, 2003). Therefore a practical use of CrPic required antidotes to reduce the level of hydroxyl radicals.

As shown in the Examples herein, the combination of CrPic, alpha-lipoic acid (α-LA), vitamin C and konjac-glucomannan (KGM) reduced levels of oxygen radicals in comparison to CrPic alone and did not nick DNA.

### Optional Components of Compositions

Comp1 and 2 may optionally include one or more additional components, such as an insulinogenic nutraceutical agent, cinnamon extract (CE) or American ginseng (AG), Uncaria tomentosa, Uncaria guianensis or Urtica dioica. Further insulinogenic nutraceutical agents include, but are not limited to, gymnema sylvestre and aloe vera.

When added to drinking water Cinnamon extract (CE; 300 mg/kg/day) improved the glucose utilization in normal male Wistar rats fed a high-fructose diet (HFD) for three weeks. HFD resulted in a 40% reduction of glucose uptake versus control, whereas CE improved glucose uptake in rats subjected to HFD diet to control levels. It has been established that the muscular insulin-stimulated IR-beta and IRS-1 tyrosine phosphorylation levels and IRS-1 associated with PI 3-kinase in HFD-fed rats are reduced -70 +/- 9%, -76 +/- 5%, and -72 +/- 6%, respectively, compared to controls (p < 0.05) and that the decreases were significantly improved by CE treatment. It is concluded that cinnamon extract reduces insulin resistance in the skeletal muscle (Quin B. et al., Horm Metab Res. 36:119-125, 2004). T2DM subjects and subjects with normal glucose tolerance exhibited up to a 19% reduction in blood glucose (area under curve) after oral glucose tolerance tests (OGTT; 25 g of glucose) following administration of AG (3g) (Vuksan et al., J Am Col Nutr. 20:370S-380S, 2001).

Uncaria tomentosa, Uncaria guianensis and Urtica dioica exhibit significant anti-inflammatory characteristics. Uncaria tomentosa and Uncaria guianensis have been reported to reduce TNF-alpha production up to 65% (Sandoval M. et al., Free Radic Biol Med. 29(1):71-8, 2000. Piscoya J. et al., Inflamm Res. 50(9):442-8, 2001). Extract of Urtica dioica at the highest concentration has been shown to inhibit LPS-induced TNF-alpha production by 79.5% and IL-1 beta production almost completely (Teucher T. et al., Arzneimittelforschung 46(9):906-10, 1996). Aqueous extract of urtica dioica (250 mg/kg) showed a strong glucose lowering effect in rats during glucose test. The decrease of glycemia reached 33+/-3.4% of the control value 1 h after glucose loading (Bnouham M. et al., Fitoterapia. 74(7-8):677-81, 2003).

### Compositions 1 and 2

Comp1 and Comp2 may comprise ingredients and ranges of quantities thereof for daily administration as provided in Table 1. Comp1 and Comp2 may comprise preferred quantities of ingredients as provided in Table 2. Magnesium salt may be selected from, but is not limited to, magnesium chloride, magnesium citrate, magnesium fumarate, magnesium malate, magnesium glutarate, and magnesium succinate. α-LA may be used preferably in the racemate form or as the R-enantiomer.

**Table 1**

| Compositions | | |
|---|---|---|
| Composition | Ingredient | Amount (Daily) |
| 1 | Mg | 1 to 3.5 g |
| 1 | α-LA | 0.8 to 1.8 g |
| 1 | Vitamin C | 0.8 to 4 g |
| 1 | KM | 3 to 8.6 g |
| 1 | CE (optional) | 2 to 5 g |
| 1 | AG (optional) | 3 to 9 g |
| 1 | Urticaria tomentosa (optional) | 0.35 to 2 g |
| 1 | Urticaria guianensis (optional) | 0.35 to 2 g |
| 1 | Urticaria dioica (optional) | 0.25 to 1 g |
| 2 | CrPic | 1 to 6 mg |
| 2 | α-LA | 0.8 to 1.8 g |
| 2 | Vitamin C | 0.8 to 4 g |
| 2 | KM | 3 to 8.6 g |
| 2 | CE (optional) | 2 to 5 g |
| 2 | AG (optional) | 3 to 9 g |
| 2 | Urticaria tomentosa (optional) | 0.35 to 2 g |
| 2 | Urticaria guianensis (optional) | 0.35 to 2 g |
| 2 | Urtica dioica (optional) | 0.25 to 1 g |

| | | |
|---|---|---|
| Mg, Magnesium Chloride; KM, Konjac mannan; CrPic, Chromium picolinate; α-LA,Alpha-lipoic acid; CE, Cinnamon extract: AG, American ginseng | | |

Mg is available from Sigma Chemicals, St. Louis, MO (*e*.*g*., as MgCl2). KM is available from Fucar International Company Ltd., Taipai, Taiwan. CrPic is available from nutrition 21, NY, USA. α-LA is available from Natrol Inc., CA, USA. CE is available as Cinnulin PF from Integrity Nutraceuticals International, FL, USA: AG is available from Superior Trading Company, CA, USA. Urticaria Tomentosa is available from Winthrow Pharmaceutical Inc., Rico Riviera, CA, USA. Urticaria guianensis is available from St. Petersburg, FL, USA. Urtica Dioica is available from VitaNet Health Food, Hartville, OH, USA.

**Table 2**

| Preferred Compositions | | |
|---|---|---|
| Composition | Ingredient | Amount (Daily) |
| 1 | Mg | 2.5 g |
| 1 | α-LA | 1.8 g |
| 1 | Vitamin C | 2 g |
| 1 | KM | 3.6 g |
| 1 | CE (optional) | 2 to 5 g |
| 1 | AG (optional) | 3 g |
| 1 | Urticaria tomentosa (optional) | 0.8 g |
| 1 | Urticaria guianensis (optional) | 0.8 g |
| 1 | Urticaria dioica (optional) | 0.5 g |
| 2 | CrPic | 3.2 mg |
| 2 | α-LA | 1.8 g |
| 2 | Vitamin C | 2 g |
| 2 | KM | 3.6 g |
| 2 | CE (optional) | 2 to 5 g |
| 2 | AG (optional) | 3 g |
| 2 | Urticaria tomentosa (optional) | 0.8 g |
| 2 | Urticaria guianensis (optional) | 0.8 g |
| 2 | Urtica dioica (optional) | 0.5 g |

### Formulation of the Novel Compositions

The active components described for use herein can be included in a pharmaceutically suitable vehicle, selected to render such compositions amenable to delivery by oral, rectal, parenteral (e.g., intravenous, intramuscular, intra-arterial, intraperitoneal, and the like), or inhalation routes, osmotic pump, and the like. Pharmaceutical compositions contemplated for use in the practice of the present invention can be used in the form of a solid, a solution, an emulsion, a dispersion, a micelle, a liposome, and the like, wherein the resulting composition contains one or more of the active compounds contemplated for use herein, as active ingredients thereof, in admixture with an organic or inorganic carrier or excipient suitable for nasal, enteral or parenteral applications. The active ingredients may be compounded, for example, with the usual non-toxic, pharmaceutically and physiologically acceptable carriers for tablets, pellets, capsules, troches, lozenges, aqueous or oily suspensions, dispersible powders or granules, suppositories, solutions, emulsions, suspensions, hard or soft capsules, caplets or syrups or elixirs and any other form suitable for use. The carriers that can be used include glucose, lactose, gum acacia, gelatin, mannitol, starch paste, magnesium trisilicate, talc, cornstarch, keratin, colloidal silica, potato starch, urea, medium chain length triglycerides, dextrans, and other carriers suitable for use in manufacturing preparations, in solid, semisolid, or liquid form. In addition auxiliary, stabilizing, thickening and coloring agents may be used.

The active compounds contemplated for use herein are included in the pharmaceutical composition in an amount sufficient to produce the desired effect upon the target process, condition or disease. In addition, such compositions may contain one or more agents selected from flavoring agents (such as peppermint, oil of wintergreen or cherry), coloring agents, preserving agents, and the like, in order to provide pharmaceutically elegant and palatable preparations. Tablets containing the active ingredients in admixture with non-toxic pharmaceutically acceptable excipients may also be manufactured by known methods. The excipients used may be, for example, (1) inert diluents, such as calcium carbonate, lactose, calcium phosphate, sodium phosphate, and the like; (2) granulating and disintegrating agents, such as corn starch, potato starch, alginic acid, and the like; (3) binding agents, such as gum tragacanth, corn starch, gelatin, acacia, and the like; and (4) lubricating agents, such as magnesium stearate, stearic acid, talc, and the like.

The tablets may be uncoated or they may be coated by known techniques to delay disintegration and absorption in the gastrointestinal tract, thereby providing sustained action over a longer period. For example, a time delay material such as glyceryl monostearate or glyceryl distearate may be employed. The tablets may also be coated by the techniques described in US Patents 4,256,108; 4,160,452; and 4,265,874, incorporated herein by this reference, to form osmotic therapeutic tablets for controlled release. When formulations for oral use are in the form of hard gelatin capsules, the active ingredients may be mixed with an inert solid diluent, for example, calcium carbonate, calcium phosphate, kaolin, or the like. They may also be in the form of soft gelatin capsules wherein the active ingredients are mixed with water or an oil medium, for an example, peanut oil, liquid paraffin, olive oil and the like.

The pharmaceutical compositions may be in the form of a sterile injectable suspension. Such a suspension may be formulated according to known methods using suitable dispersing or wetting agents and suspending agents. The sterile injectable preparation may also be a sterile injectable solution or suspension in a non-toxic parenterally-acceptable diluent or solvent, for example, as a solution in 1,4-butanediol. Sterile, fixed oils are conventionally employed as a solvent or suspending medium. For this purpose, any bland fixed oil may be employed including synthetic mono- or diglycerides, fatty acids (including oleic acid), naturally occurring vegetable oils like sesame oil, coconut oil, peanut oil, cottonseed oil, etc., or synthetic fatty vehicles like ethyl oleate or the like.

Buffers, preservatives, antioxidants, and the like can be incorporated as required. Compositions contemplated for use in the practice of the present invention may also be administered in the form of suppositories for rectal administration of the active ingredients. These compositions may be prepared by mixing the active ingredients with a suitable non-irritating excipient, such as cocoa butter, synthetic glyceride esters of polyethylene glycols (which are solid at ordinary temperatures, but liquefy and/or dissolve in the rectal cavity to release the active ingredients), and the like. In addition, sustained release systems, including semi-permeable polymer matrices in the form of shaped articles (e.g., films or microcapsules) can also be used for the administration of the active compounds employed herein.

The following Examples are offered by way of illustration and not by way of limitation.

### EXAMPLES

### EXAMPLE 1

Fatty diabetic Zucker rats were separated into three groups to receive three diets for 4 weeks: a) 1% Mg in diet (5 times higher than control diet); b) a combination including 2 g/L of vitamin C in drinking water, 2.5% of KM in diet, 400 mg/kg of α-LA; c) Comp1 that includes 1% of Mg in diet, 2 g/L of vitamin C in drinking water, 2.5% of KM in diet, 400 mg/kg of α-LA.

No change in the level of fasting glucose level vs baseline was observed by the administration of Mg. 17% reduction of glucose vs baseline was observed by the combination of Vitamin C, KM and α-LA. 28% reduction of glucose vs baseline was observed by Comp1 (the combination of Vitamin C, KM and α-LA with Mg salt)

### EXAMPLE 2

### DNA CLEAVAGE REACTION

The reaction is carried out by mixing aliquots of pUC19 [in 5 mM Tris and 500 µM EDTA buffer (pH 8.0)], reductant in H₂O, CrPic in H₂O, phosphate buffer (pH 7.0) and H₂O to give a final volume of 15 µL.; the final phosphate buffer concentration was always 50 mM. Inhibitors were added as solutions in H2O, as solutions in phosphate buffer or as neat liquids. Reactions were allowed to proceed for 20 min. unless otherwise noted. All reactions were quenched by the addition of loading buffer (24% glycerol and 0.1% bromophenol blue). Aliquots (7 µl) were directly loaded onto 1% agarose gel and were electrophoresed at 70V. The gel was stained with ethidium bromide - and was photographed on a UV transilluminator. Any timed assays were performed by adding the appropriate reagents to the sample tubes, and the addition of DNA was marked as time zero. The reactions were quenched at various time points by adding loading buffer. Reactions under argon were performed by bubbling the gas through the reaction mixture. All the reagents were added and the container was sealed until the reaction was quenched by addition of loading buffer.

### EXAMPLE 3

120 nM (49 µg) of CrPic nick DNA in the conditions of increase in the level of ascorbate or excess of hydrogen peroxide (J. B. Vincent Sports' Med. 2003, 33, 3, 213-230)

Effect of the Comp2 (Cr Pic 400 µg, Vitamin C 2 g, α-LA 1.8 g and KM 3.6 g) on DNA was studied in the conditions of increase in the level of ascorbate or excess of hydrogen peroxide. No effect of Comp2 on DNA was found.

## Claims

1. Use of Comp1 for the manufacture of a medicament for the treatment and/or prevention of diabetes or diabetes related diseases selected from the group consisting of hyperglycemia, hyperinsulinemia, insulin resistance, inflammation or side effects of diabetes in mammals, selected from nephropathy, cardiomyopathy, stroke and blindness, or for the reduction of blood glucose levels in mammals, wherein Comp1 comprises magnesium salt, konjac mannan, vitamin C and alpha-lipoic acid.

2. Use according to claim 1 wherein Comyl potentiates hypoglycemic effects of a magnesium salt to effectively treat and/or prevent diabetes.

3. Use according to any one of the preceding claims, wherein the amount of ingredients of Comp1 administered comprises: magnesium chloride (1 to 3.6 g/day), alpha-lipoic acid (0.8 to 1.8 g/day), konjac mannan (3 to 8.6 g/day), vitamin C (0.8 to 4 g/day).

4. Use according to any one of the preceding claims, wherein the amount of ingredients of Comp1 administered comprises: magnesium chloride (2.5 g/day), alpha-lipoic acid (1.8 g/day), konjac mannan (3.6 g/day), vitamin C (2 g/day).

5. Use according to any one of the preceding claims, wherein the administration of the medicament is oral, parenteral, rectal, sublingual or inhalation administration.

6. Use according to any one of the preceding claims, wherein Comp1 further comprises a physiologically acceptable carrier suitable for oral, parenteral, rectal, sublingual or inhalation administration.

7. Use according to any one of the preceding claims, wherein the disease related to diabetes is hyperglycemia.

8. Comp1 for use in the prevention and/or treatment of diabetes or diabetes related diseases selected from the group consisting of hyperglycemia, hyperinsulinemia, insulin resistance, inflammation or side effects of diabetes in mammals, selected from neuropathy, nephropaty, cardiomyopathy, stroke and blindness, or for use in the reduction of blood glucose levels in mammals wherein Comp1 comprises magnesium salt, konjac mannan, vitamin C and alpha-lipoic.

9. Comp1 for use according to claim 8, wherein the amount of ingredients of Comp1 administered comprises: magnesium chloride (1 to 3.5 g/day), alpha-lipoic acid (0.8 to 1.8 g/day), konjac mannan (3 to 8.6 g/day), vitamin C (0.8 to 4 g/day).

10. Comp1 for use according to any one of claims 8 or 9, wherein the administration of Comp1 is oral, parenteral, rectal, sublingual or inhalation administration.

11. Comp1 for use according to any one of claims 8 to 10, wherein Comp1 further comprises a physiologically acceptable carrier suitable for oral, parenteral, rectal, sublingual or inhalation administration.

12. Comp 1 for use according to any one of claims 8 to 11, wherein the diabetes related disease is hyperglycemia.

13. Comp1 wherein Comp1 comprises magnesium salt, konjac mannan, vitamin C and alpha-lipoic acid.

14. Comp1 according to claim 13, wherein Comp1 further comprises a physiologically acceptable carrier suitable for oral, parenteral, rectal, sublingual or inhalation administration.

## Patentansprüche

1. Verwendung der Zusammensetzung 1 zur Herstellung eines Medikaments zur Behandlung und/oder Vorbeugung von Diabetes oder Krankheiten im Zusammenhang mit Diabetes, ausgewählt aus Gruppe bestehend aus Hyperglykämie, Hyperinsulinämie, Insulinresistenz, Entzündung, oder Nebenwirkungen von Diabetes bei Säugern, ausgewählt aus Neuropathie, Nephropathie, Kardiomyopathie, Schlaganfall und Erblindung, oder zur Reduktion von Blutglukosespiegeln bei Säugern, wobei Zusammensetzung 1 Magnesiumsalz, Konjac-Mannan, Vitamin C und alpha-Liponsäure umfasst.

2. Verwendung nach Anspruch 1, wobei Zusammensetzung 1 die blutdrucksenkenden Wirkungen eines Magnesiumsalzes zur effektiven Behandlung und/oder Vorbeugung von Diabetes verstärkt.

3. Verwendung nach einem der vorhergehenden Ansprüche, wobei die verabreichte Menge der Inhaltsstoffe der Zusammensetzung 1 umfasst: Magnesiumchlorid (1 bis 3,5 g/Tag), alpha-Liponsäure (0,8 bis 1,8 g/Tag), Konjac-Mannan (3 bis 8,6 g/Tag), Vitamin C (0,8 bis 4 g/Tag) :

4. Verwendung nach einem der vorhergehenden Ansprüche, wobei die verabreichte Menge der Inhaltsstoffe der Zusammensetzung 1 umfasst: Magnesiumchlorid (2,5 g/Tag), alpha-Liponsäure (1,8 g/Tag), Konjac-Mannan (3,6 g/Tag), Vitamin C (2 g/Tag).

5. Verwendung nach einem der vorhergehenden Ansprüche, wobei es sich bei der Verabreichung des Medikaments um orale, parenterale, rektale, sublinguale oder inhalative Verabreichung handelt.

6. Verwendung nach einem der vorhergehenden Ansprüche, wobei die Zusammensetzung 1 darüber hinaus einen physiologisch annehmbaren Träger umfasst, der für eine orale, parenterale, rektale, sublinguale oder inhalative Verabreichung geeignet ist.

7. Verwendung nach einem der vorhergehenden Ansprüche, wobei es sich bei der Krankheit im Zusammenhang mit Diabetes um Hyperglykämie handelt.

8. Zusammensetzung 1 zur Verwendung zur Vorbeugung und/oder Behandlung von Diabetes oder Krankheiten im Zusammenhang mit Diabetes, ausgewählt aus der Gruppe bestehend aus Hyperglykämie, Hyperinsulinämie, Insulinresistenz, Entzündung, oder Nebenwirkungen von Diabetes bei Säugern, ausgewählt aus Neuropathie, Nephropathie, Kardiomyopathie, Schlaganfall und Erblindung, oder zur Verwendung zur Reduktion von Blutglukosespiegeln bei Säugern, wobei Zusammensetzung 1 Magnesiumsalz, Konjac-Mannan, Vitamin C und alpha-Liponsäure umfasst.

9. Zusammensetzung 1 zur Verwendung nach Anspruch 8, wobei die verabreichte Menge der Inhaltsstoffe der Zusammensetzung 1 umfasst: Magnesiumchlorid (1 bis 3,5 g/Tag), alpha-Liponsäure (0,8 bis 1,8 g/Tag), Konjac-Mannan (3 bis 8,6 g/Tag), Vitamin C (0,8 bis 4 g/Tag).

10. Zusammensetzung 1 zur Verwendung nach einem der Ansprüche 8 oder 9, wobei es sich bei der Verabreichung der Zusammensetzung 1 um orale, parenterale, rektale, sublinguale oder inhalative Verabreichung handelt.

11. Zusammensetzung 1 zur Verwendung nach einem der Ansprüche 8 bis 10, wobei die Zusammensetzung 1 darüber hinaus einen physiologisch annehmbaren Träger umfasst, der für eine orale, parenterale, rektale, sublinguale oder inhalative Verabreichung geeignet ist.

12. Zusammensetzung 1 zur Verwendung nach einem der Ansprüche 8 bis 11, wobei es sich bei der Krankheit im Zusammenhang mit Diabetes um Hyperglykämie handelt.

13. Zusammensetzung 1, wobei Zusammensetzung 1 Magnesiumsalz, Konjac-Mannan, Vitamin C und alpha-Liponsäure umfasst.

14. Zusammensetzung 1 nach Anspruch 13, wobei die Zusammensetzung 1 darüber hinaus einen physiologisch annehmbaren Träger umfasst, der für eine orale, parenterale, rektale, sublinguale oder inhalative Verabreichung geeignet ist.

## Revendications

1. Utilisation d'une composition "Comp1" pour la fabrication d'un médicament conçu pour traiter et/ou prévenir, chez des mammifères, le diabète ou une maladie liée au diabète, choisie parmi hyperglycémie, hyperinsulinémie, résistance à l'insuline et inflammation, ou un effet secondaire du diabète, choisi parmi neuropathie, néphropathie, cardiomyopathie, attaque et cécité, ou pour faire baisser le taux de glucose sanguin chez des mammifères, laquelle composition "Comp1" comprend un sel de magnésium, du mannane de konjac, de la vitamine C et de l'acide alpha-lipoïque.

2. Utilisation conforme à la revendication 1, dans laquelle la composition "Comp1" renforce les effets hypoglycémiants d'un sel de magnésium, pour un traitement et/ou une prévention efficace(s) du diabète.

3. Utilisation conforme à l'une des revendications précédentes, dans laquelle les quantités des ingrédients de Comp1 à administrer sont les suivantes : 1 à 3,5 g/jour de chlorure de magnésium, 0,8 à 1,8 g/jour d'acide alpha-lipoïque, 3 à 8,6 g/jour de mannane de konjac, et 0,8 à 4 g/jour de vitamine C.

4. Utilisation conforme à l'une des revendications précédentes, dans laquelle les quantités des ingrédients de Comp1 à administrer sont les suivantes : 2,5 g/jour de chlorure de magnésium, 1,8 g/jour d'acide alpha-lipoïque, 3,6 g/jour de mannane de konjac, et 2 g/jour de vitamine C.

5. Utilisation conforme à l'une des revendications précédentes, dans laquelle l'administration du médicament est une administration par voie orale, parentérale, rectale ou sublinguale ou par inhalation.

6. Utilisation conforme à l'une des revendications précédentes, dans laquelle la composition Comp1 comprend en outre un véhicule physiologiquement admissible, approprié pour administration par voie orale, parentérale, rectale ou sublinguale ou par inhalation.

7. Utilisation conforme à l'une des revendications précédentes, la maladie liée au diabète étant l'hyperglycémie.

8. Composition "Comp1" pour utilisation dans la prévention et/ou le traitement, chez des mammifères, du diabète ou d'une maladie liée au diabète, choisie parmi hyperglycémie, hyperinsulinémie, résistance à l'insuline et inflammation, ou d'un effet secondaire du diabète, choisi parmi neuropathie, néphropathie, cardiomyopathie, attaque et cécité, ou pour utilisation dans le but de faire baisser le taux de glucose sanguin chez des mammifères, laquelle composition "Comp1" comprend un sel de magnésium, du mannane de konjac, de la vitamine C et de l'acide alpha-lipoïque.

9. Composition "Comp1" pour utilisation conforme à la revendication 8, dans laquelle les quantités des ingrédients de Comp1 à administrer sont les suivantes : 1 à 3,5 g/jour de chlorure de magnésium, 0,8 à 1,8 g/jour d'acide alpha-lipoïque, 3 à 8,6 g/jour de mannane de konjac, et 0,8 à 4 g/jour de vitamine C.

10. Composition "Comp1" pour utilisation conforme à l'une des revendications 8 et 9, de laquelle composition Comp1 l'administration est une administration par voie orale, parentérale, rectale ou sublinguale ou par inhalation.

11. Composition "Comp1" pour utilisation conforme à l'une des revendications 8 à 10, laquelle composition Comp1 comprend en outre un véhicule physiologiquement admissible, approprié pour administration par voie orale, parentérale, rectale ou sublinguale ou par inhalation.

12. Composition "Comp1" pour utilisation conforme à l'une des revendications 8 à 11, la maladie liée au diabète étant l'hyperglycémie.

13. Composition "Comp1", laquelle composition "Comp1" comprend un sel de magnésium, du mannane de konjac, de la vitamine C et de l'acide alpha-lipoïque.

14. Composition "Comp1" conforme à la revendication 13, laquelle composition "Comp1" comprend en outre un véhicule physiologiquement admissible, approprié pour administration par voie orale, parentérale, rectale ou sublinguale ou par inhalation.
